(19) Europäisches Patentamt
European Patent Office
Office européen des brevets

(11) **EP 3 628 266 B1**

(12) **EUROPÄISCHE PATENTSCHRIFT**

(45) Veröffentlichungstag und Bekanntmachung des Hinweises auf die Patenterteilung:
**21.10.2020 Patentblatt 2020/43**

(51) Int Cl.:
**A61C 1/00** (2006.01)     **A61N 5/06** (2006.01)
A61B 18/20 (2006.01)     A61N 5/067 (2006.01)

(21) Anmeldenummer: **18197486.6**

(22) Anmeldetag: **28.09.2018**

(54) **BAKTERIENENTFERNUNGSLASER**

BACTERIA REMOVAL LASER

LASER DESTINÉ À L'ÉLIMINATION DES BACTÉRIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.04.2020 Patentblatt 2020/14**

(73) Patentinhaber: **Ivoclar Vivadent AG**
**9494 Schaan (LI)**

(72) Erfinder: **Reinhardt, Jonas**
**7206 Igis (CH)**

(74) Vertreter: **Baronetzky, Klaus**
**Splanemann**
**Patentanwälte Partnerschaft**
**Rumfordstrasse 7**
**80469 München (DE)**

(56) Entgegenhaltungen:
**EP-A2- 1 591 076**     **EP-A2- 3 222 243**
**US-A1- 2012 015 318**

**Beschreibung**

Die Erfindung betrifft einen Bakterienentfernungslaser, insbesondere zur Entfernung von Kariesbakterien an natürlichen oder prothetischen Zähnen.

Für die Bekämpfung von Bakterien, die Hartgewebe und/oder Weichgewebe eines Menschen oder Tieres befallen haben, ist es seit langem bekannt, bakteriozide Substanzen einzusetzen.

Hierzu zählen insbesondere Antibiotika. Abgesehen davon, dass durch Antibiotikagabe Resistenzen entstehen können, sind Antibiotika ungeeignet oder wenig wirksam, wenn tiefliegende Gewebeschichten betroffen sind. Dies gilt insbesondere für Hartgewebe.

Zu den Hartgeweben gehören vor allem die menschlichen und tierischen Zähne. Diese sind häufig von Zahnkaries mit Kariesbakterien befallen, zu denen insbesondere Streptococcus mutans gehört.

Typischerweise wird bei Zahnkaries so verfahren, dass kariöses Zahnmaterial mittels eines üblichen Zahnbohrers entfernt wird.

Durch das Entfernen des Zahnhartgewebes wird der Zahn geschädigt. Dennoch ist es erforderlich, vorsorglich etwas mehr als das befallene kariöse Zahnmaterial zu entfernen, um sicherzustellen, dass keine Kariesbakterien unter der Dentalrestauration, die der Zahnarzt anschließend herstellt, verbleiben.

Zur Vermeidung von Karies sind auch andere Maßnahmen bekannt geworden. Hierzu gehört beispielsweise die Fluoridierung mit speziellen Fluoridpräparaten und die Kariesinfiltration. Bei dieser wird niedrigviskoser Kunststoff in das kariotische Zahngewebe eingebracht und soll so eine Diffusionssperre bilden.

Ferner ist die Abdeckung mit Calciumhydroxyd oder Glasionomerzement bekannt geworden.

Ferner ist auch die Verwendung von Laserlicht zur Kariesbehandlung seit mehr als 20 Jahren bekannt. Gemäß der EP 1 052 947 A1 sollen Kariesbakterien mittels eines Nd:YAG-Lasers verdampft werden. Um die erwünschte Wirkung zu erzielen, werden Laserleistungen von 1 bis 10 Watt eingesetzt, so dass eine intensive Bestrahlung der Behandlungsoberfläche mittels der Laserstrahlung erfolgen kann. Keine Tiefenwirkung.

Die Erprobung dieser Technik in den letzten 20 Jahren hat jedoch ergeben, dass sie keine langfristigen Erfolge hat. Während die Ausbreitung der Karies oberflächlich gestoppt erscheint, breitet sich die Karies bei der Verwendung dieser Technik langfristig weiter aus, so dass über kurz oder lang radikalere Maßnahmen wie der Ersatz der betroffenen Zahnhartsubstanz durch eine Zahnfüllung, ein Onlay oder eine Überkronung oder eine Wurzelkanalbehandlung erforderlich sind.

Wenn diese Maßnahmen nicht ergriffen werden, besteht die Gefahr, dass der betreffende Nachbarzahn und gegebenenfalls der Antagonist ebenfalls kariotisch werden.

US 2012/0015318 A1 offenbart ein Verfahren zur Ablation von kariöser Zahnsubstanz unter Verwendung von Laserstrahlung mit einer Wellenlänge von beispielsweise 2500 nm oder 2940 nm.

Demgegenüber liegt der Erfindung die Aufgabe zu Grunde, einen Bakterienentfernungslaser gemäß dem Oberbegriff von Anspruch 1 zu schaffen, welcher langfristig wirkt.

Diese Aufgabe wird erfindungsgemäß durch Anspruch 1 gelöst. Vorteilhafte Weiterbildungen ergeben sich aus den Unteransprüchen.

Erfindungsgemäß ist der Bakterienentfernungslaser als Handgerät ausgebildet. Er weist dementsprechend eine Handhabe auf, über welche die Richtung und die Raumpositionen der Laser-Strahlungsemission der Laser-Strahlungsquelle gesteuert werden kann.

Die Laser-Strahlungsquelle kann ebenfalls in dem Handstück sein, oder an einer ortsfesten Basisstation, die dann mit einem gegebenenfalls flexiblen Strahlungs-Leitstab mit der Greifhandhabe verbunden ist. Die Greifhandhabe weist auch eine Strahlungsaustrittsfläche auf, an welcher Stelle die von der Laser-Strahlungsquelle emittierte Strahlung den Laser verlässt.

Dem Strahlungs-Leitstab vorgeschaltet oder nachgeschaltet oder beidseitig ist auch eine Beaufschlagungsoptik vorgesehen. Diese kann beispielsweise der Strahlungs-Vergleichmäßigung dienen, oder bei Bedarf der Bündelung der abgegebenen Strahlung.

Der Laser emittiert im Wellenlängenbereich zwischen 2200 nm und 4000 nm. Insofern geeignet ist ein Er:YAG-Laser, mit einem Emissionsmaximum von ca. 2940 nm.

Alternativ sind auch GaInAsSb-Laser, GaInSn-Laser oder GaInSb/GaSb-Laser möglich, die zwischen 2100 nm und 4000 nm emittieren.

Der Laser kann auch mittels eines Mikrowellenstrahlers, z.B. im Frequenzbereich zwischen 2 und 3 GHz arbeiten und bildet dann einen Maser.

Laser mit einer großen Wellenlänge, also im langen Infrarotbereich, werden typischerweise für die Anwendung am menschlichen Körper nicht empfohlen, da beispielsweise Haut bei einer intensiven Laserlicht-Beaufschlagung bei dieser Wellenlänge zur Verbrennung neigt.

Zudem wird in der gängigen Fachliteratur darauf hingewiesen, dass Laserstrahlung mit einer größeren Wellenlänge eine geringere Eindringtiefe habe, aufgrund der dort bestehenden Absorptionsmaxima von Wasser.

Erfindungsgemäß weist der Bakterienentfernungslaser jedoch eine solche Wellenlänge auf und eine über die Zeit gemittelte Energieabgabe von weniger als 1 mJ/mm$^2$.

Überraschend ergibt sich mit einer solchen Laserbeaufschlagung eine nachhaltige Bakterienentfernung. Die Beaufschlagungszeit beträgt für eine entsprechend hohe Eindringtiefe mindestens mehrere Minuten, bevorzugt 10 bis 15 Minuten.

Die hohe Absorptionsfähigkeit von Wasser bei ca. 3000 nm wird ausgenutzt. Durch die Laserbeaufschlagung verdunstet das Wasser - bei Temperaturen deutlich unter 100 Grad Celsius - an der Behandlungsfläche und unterhalb dieser.

Die dort befindlichen Bakterien werden offenbar mitverdampft oder zumindest geschädigt.

Wenn das Wasser der ersten Schicht mit beispielsweise einer Schichtstärke von 1 mm verdampft ist, dringt die nunmehr nicht mehr durch Wasser absorbierte Laserstrahlung tiefer ein und wirkt auf den nächsten Millimeter.

Auf diese Art erfolgt die Beaufschlagung mit der erfindungsgemäßen Laserstrahlung zur Bakterienentfernung so lange, bis auch die Kariesbakterien geschädigt sind, die in tieferen Schichten unterhalb der Behandlungsfläche leben.

Erfindungsgemäß ist es vorteilhaft, die Energiedichte und die Leistung des Bakterienentfernungslasers so zu wählen, dass lediglich eine Temperaturerhöhung am Zahn von wenigen Grad, beispielsweise maximal 8 oder höchstens 15 Grad stattfindet. Untersuchungen haben ergeben, dass eine langsame Temperaturerhöhung entscheidend ist, um die Gefahr von Spannungsrissen in der Zahnoberfläche zu beseitigen. Bevorzugt beträgt die Temperaturerhöhung weniger als 20 Grad/min, besonders bevorzugt weniger als 15 Grad/min.

Die angestrebte Maximaltemperatur der Behandlungsoberfläche beträgt 45 Grad Celsius. Hierbei wirkt sich die Auswahl eines geeigneten Impuls/Pausen-Verhältnisses günstig aus. Beispielsweise kann das Impuls/Pausen-Verhältnis 1 zu 20 oder 1 zu 30 betragen. Dies stellt einen insofern günstigen Kompromiss dar.

Erfindungsgemäß reicht es aus, wenn die Bakterien so weit geschädigt werden, dass sie nicht mehr fortpflanzungsfähig sind und keine "Säure / Stoffwechselprodukte" mehr produzieren können.

Es ist nicht erforderlich, durch den Dampfdruck die Zellwände zu zerstören, also durch Verdampfung bei 100 Grad Celsius diese platzen zu lassen, sondern es reicht eine Erwärmung auf eine wesentlich geringere Temperatur aus, ähnlich dem Pasteurisieren.

Der erfindungsgemäße Einsatz des Bakterienentfernungslasers vermeidet auch die Notwendigkeit, ein separates Kühlmittel zu verwenden. Damit ist die Gefahr von thermischen Spannungen und Mikrorissen in der Zahnoberfläche eliminiert. Der Verzicht auf eine Wasserkühlung erlaubt eine hohe Mobilität des Lasers. Auch wenn Dentalprothesen selbstverständlich nie kariotisch sind, lässt sich der erfindungsgemäße Bakterienentfernungslaser auch zur "Sterilisierung" dieser einsetzen, denn auch die dort befindlichen Bakterien - die ebenfalls beispielsweise Streptococcus mutans sein können - lassen sich erfindungsgemäß schädigen.

Dies ist besonders zu empfehlen bei Teilprothesen, weil bei diesen die Mundhygiene besonders wichtig ist.

Ein besonderer Vorteil der Verwendung der Laserstrahlung mit etwa 3000 Nanometer ist, dass sowohl Dentin als auch Zahnschmelz für sich genommen weitgehend transparent für diese Wellenlänge sind. Dies gilt selbstverständlich nicht für feuchtes Zahnhartgewebe. Wasser und damit auch Bakterien absorbieren bei der genannten Wellenlänge besonders gut.

Die Kombination dieser Merkmale lässt sich dadurch ausnutzen, dass auch tiefliegende Bakterien erfindungsgemäß gut erreichbar sind, durch längere Einwirkungszeit des Lasers.

Eingeleitete Laserstrahlung wird mit zunehmender Eindringtiefe schwächer, wenn Wasser und Bakterien an der betreffenden Stelle vorhanden sind. Die Absorption erfolgt beispielsweise mit 15% bis 25% pro mm, oder aber z.B. mit 20% bis 35% pro mm Eindringtiefe.

Mit zunehmender Bakterienentfernung und Trocknung des Zahnhartgewebes nimmt die Eindringtiefe jedoch zu, so dass der Bereich, in dem sich die Laserstrahlung abschwächt, weiter zum Zahninneren, also zum Dentin hin verlagert wird.

Durch diesen gewünschten Effekt lässt sich eine Entfernung auch tiefliegender Bakterien sicherstellen.

Überraschend lassen sich insofern auch diese tiefliegenden Bakterien unschädlich machen.

Vorteilhaft ist es, wenn der Bakterienentfernungslaser einen Sensor aufweist, der dafür bestimmt ist, die von der Behandlungsfläche reflektierte Strahlung oder die durch die Behandlungsfläche und die darunterliegenden Bereiche durchgelassene Strahlung zu erfassen.

Es ist auch möglich, beide Arten der Erfassung zu kombinieren.

Gerade ein Transmissionssensor ist in der Lage zu erfassen, welche Bestrahlung die Behandlungsfläche und die darunterliegenden Bereiche, also die Behandlungsbereiche, durchtritt.

Beispielsweise kann aus der Zunahme des Ausgangssignals des Sensors geschlossen werden, dass hinreichend Feuchtigkeit und Bakterien entfernt worden sind, so dass die Behandlung abgeschlossen werden kann.

Es ist auch möglich, in Strahlungspausen der getakteten Laserstrahlung zu messen. In diesem Fall erfasst der Sensor die Emission des Behandlungsbereiches in einem geeigneten Wellenlängenbereich zwischen 1000 und 14000 nm, z.B. bei 3000 nm, insofern also die Temperatur, beispielsweise des Zahns.

Bevorzugt wird die Temperatur während der Bestrahlung gemessen, und zwar vorteilhafterweise in den Wellenlängenbereichen 1 - 2,8 Mikrometer oder von 3,5 - 14 Mikrometer, also außerhalb des Emissionsmaximums des Er:YAG-Lasers.

Bevorzugt weist der Bakterienentfernungslaser eine Steuervorrichtung auf. Diese dient dazu, den Laser einzuschalten und entweder für eine vorgegebene, aber bevorzugt wählbare Zeit strahlen zu lassen, oder aber den Laser auszuschalten, wenn die Kariesbakterienentfernung abgeschlossen ist.

In Verbindung mit dem Sensor kann aber auch beispielsweise die Strahlung reduziert werden, wenn sich ergibt, dass der Behandlungsbereich zu warm geworden ist. In diesem Fall wird die Behandlung bevorzugt automatisch verlängert.

Die Strahlungsaustrittsfläche ist bevorzugt kleiner als ein Zahn. Sie kann zwischen 1 und 7 mm Durchmesser haben, ist bevorzugt kreisförmig und hat bevorzugt einen Durchmesser um 4 mm.

Bevorzugt ist der erfindungsgemäße Laser mit einer Sterilwasserquelle gekoppelt. Durch diese lässt sich der Behandlungsbereich nach der Behandlung spülen und zugleich auch benetzen, so dass eine versehentliche Übertrocknung des Behandlungsbereichs vermeidbar ist. Die Sterilwasserquelle kann auch nachgelagert eingesetzt werden, wobei sie dem Laser, auch wenn sie dort nicht eingebaut ist, jedenfalls funktional zugeordnet ist.

Offenbart ist auch ein nicht erfindungsgemäßes System aus einem Bakterienentfernungslaser und einer Behandlungsfläche dar. Es ist dabei vorgesehen, dass der Laser Strahlung im Wellenlängenbereich zwischen 1500 und 4500 nm, insbesondere oberhalb von 2200 nm abgibt und der Behandlungsfläche zuleitet.

Während des Beginns der Strahlungsabgabe wird die Laserstrahlung zu 20 bis 35 % pro mm Eindringtiefe absorbiert. Sie schädigt damit Bakterien oder vernichtet diese.

Bei einer Ausführungsform des o.g. nicht erfindungsgemäßen Systems gelangt die Strahlung mit einer über die Zeit gemittelten Energiedichte zwischen 0,01 mJ/mm$^2$ und 10 mJ/mm$^2$ auf die Behandlungsfläche.

Die Erfindung ermöglicht die Verwendung eines Bakterienentfernungslasers zur Entfernung von Kariesbakterien, insbesondere zur Entfernung deren Stoffwechsels, deren Erbguts und/oder zur letalen Entfernung dieser.

Weitere Vorteile, Einzelheiten und Merkmale ergeben sich aus der nachfolgenden Beschreibung eines Ausführungsbeispiels der Erfindung anhand der Zeichnung.

Es zeigen:

Fig. 1  einen erfindungsgemäßen Bakterienentfernungslaser in schematischer perspektivischer Darstellung, in Aktion zur Entfernung von Kariesbakterien in einer ersten Ausführungsform;

Fig. 2  eine weitere Ausführungsform eines erfindungsgemäßen Bakterienentfernungslasers mit Sensor;

Fig. 3  eine dritte Ausführungsform eines erfindungsgemäßen Bakterienentfernungslasers;

Fig. 4  eine schematische Darstellung eines Hartgewebes mit angedeuteten Bakterien;

Fig. 5  eine Darstellung der zunehmenden Eindringtiefe der Laserstrahlung in das Hartgewebe;

Fig. 6  ein Diagramm zur gemessenen Temperaturerhöhung bei nassen im Vergleich zu trockenen Zähnen; und

Fig. 7  eine Darstellung der vom Temperatursensor gemessenen Temperatur eines nassen Zahns sowie eines trockenen Zahns.

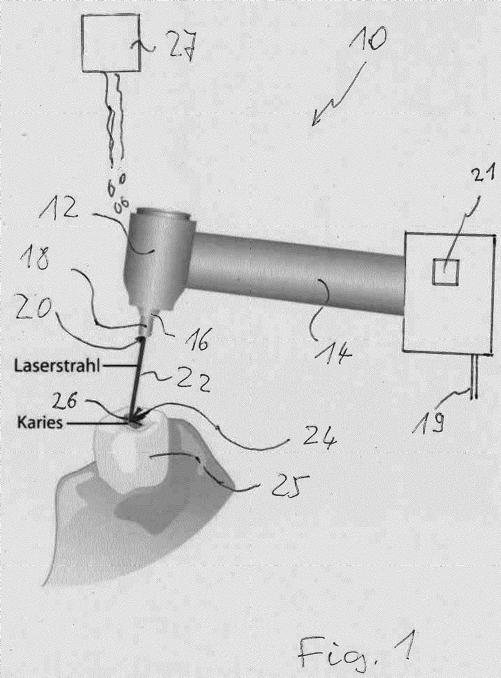

Aus Fig. 1 ist ein Bakterienentfernungslaser in einer ersten Ausführungsform ersichtlich. Der Bakterienentfernungslaser 10 weist eine Strahlungsquelle 12 auf, die in einer Basisstation aufgenommen ist und über einen insbesondere auch wärmedurchlässigen Lichtleiter mit einem Handgerät verbunden ist. Ggf. könnte auch alles im Kopfbereich eines Handgerätes realisiert sein. Das Handgerät weist eine Greifhandhabe 14 auf, über die ein Bediener es in die gewünschte Position bringen kann.

Im Bereich des Kopfes des Handgeräts ist eine Beaufschlagungsoptik 16 angeordnet, die vom Laserstrahl, den die Strahlungsquelle 12 erzeugt, durchtreten wird.

Es ist ein Strahlungsleitstab 18 angeordnet, der an einer Strahlungsaustrittsfläche 20 endet.

Das Handgerät weist einen Netzanschluss 19 auf, der die Energiequelle bildet, sowie eine Steuervorrichtung 21.

Die Strahlungsquelle ist als Er:YAG-Laser ausgebildet und emittiert Laserstrahlung mit einem Emissionsmaximum von 2940 Nanometern.

Die Laserstrahlung 22 trifft auf eine Behandlungsfläche 24. Diese ist im vorliegenden Ausführungsbeispiel an einem Molar 25 ausgebildet, dessen okklusale Fläche mit Karies 26 befallen ist.

Die Behandlungsfläche ist in diesem Ausführungsbeispiel eine Kreisfläche, die einen Durchmesser von einem Millimeter hat. Es versteht sich, dass die Größe dieser Fläche in weiten Bereichen an die Erfordernisse angepasst werden kann. Beispielsweise kann der Durchmesser auch durchaus 5 mm betragen.

Die Größe der Behandlungsfläche lässt sich auch einstellbar machen. Hierzu weist die Beaufschlagungsoptik 16 eine entsprechende Verstellmöglichkeit auf. Bevorzugt ist die Größe der Behandlungsfläche so groß wie der typischerweise von Karies befallene Bereich, also beispielsweise 3 mm Durchmesser. Wenn der Durchmesser kleiner als der von Karies befallene Bereich ist, muss der Bediener die Behandlungsfläche über den gesamten von Karies befallenen Bereich bewegen, so dass die Behandlung verlängert wird.

Erfindungsgemäß überprüft der Bediener des erfindungsgemäßen Lasers zunächst, welche Bereiche des Zahnes 25 von Karies befallen sind. Er richtet in an sich bekannter Weise, also, wie es bei Lichthärtegeräten üblich ist, die Laserstrahlung auf einen solchen Bereich und schaltet das Handgerät ein.

Die Laserstrahlung wird gepulst mit einem Impulspausenverhältnis von 1:30 abgegeben.

Es wird Laserstrahlung mit einer Energiedichte kleiner als 1 mJ/mm$^2$ über die Zeit gemittelt abgegeben. Diese trifft auf die Behandlungsfläche 24.

Der Bediener hält diese Position für mehrere Minuten aufrecht.

Die gepulste Laserstrahlung schädigt die Kariesbakterien im Bereich der Behandlungsfläche 24. Zunächst wird der oberflächliche Bereich der Behandlungsfläche, also knapp unter der Oberfläche, schwerpunktmäßig beaufschlagt. Die dort vorhandene Flüssigkeit, im Wesentlichen Speichel, verdunstet, so dass die Laserstrahlung tiefer eindringt. Je nach erwünschter Eindringtiefe ist es auch möglich, die Strahlungsbeaufschlagung für 15 oder gar 20 Minuten aufrechtzuerhalten.

Unmittelbar unterhalb der Behandlungsfläche 24 weist der Zahn 25 Zahnschmelz auf. In diesen sind Kariesbakterien eingedrungen, und zwar typischerweise 1 bis 3 mm, wo sie ohne den erfindungsgemäßen Bakterienentfernungslaser 10 Karies auslösen würden.

In einer vorteilhaften Ausgestaltung der Erfindung ist es vorgesehen, den Bakterienentfernungslaser mit einer Stützvorrichtung am Kiefer oder Kinn des Patienten abzustützen.

Die nicht dargestellte Stützvorrichtung ist einstellbar und erlaubt es, eine Position vorzugeben, an welcher der Laserstrahl 22 auf die erwünschte Behandlungsfläche 24 trifft.

Mit der Stützvorrichtung lässt sich auch der Abstand zwischen der Strahlungsaustrittsfläche 20 und der Behandlungsfläche 24 einstellen. Dieser beträgt bevorzugt zwischen 1 mm und 2 cm.

Nach Abschluss der Laserbehandlung wird der ursprünglich von Kariesbakterien befallene Bereich durch die Sterilwasserquelle 27 bevorzugt mit sterilem Wasser gespült. Hierdurch wird zum einen der Speichelverlust während der Behandlung kompensiert und zum anderen werden tote Bakterien weggespült.

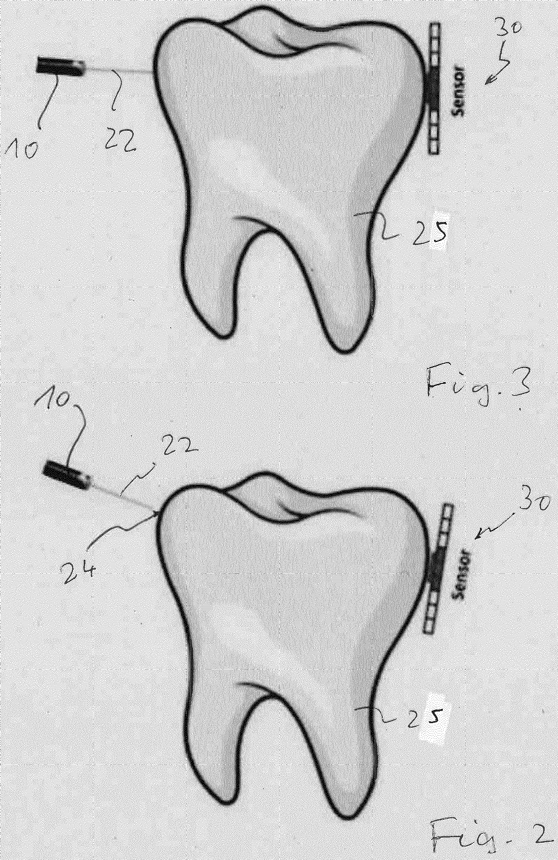

Fig. 2 zeigt eine weitere Ausführungsform eines erfindungsgemäßen Bakterienentfernungslasers. Bei dieser ist an der dem Laser 10 gegenüberliegenden Seite eines Zahns 25 ein Sensor 30 angebracht. Der Sensor erfasst die den Zahn 25 durchtretende Laserstrahlung. Insbesondere wird eine Veränderung der Laserstrahlung erfasst.

Nachdem die Laserstrahlung im Bereich der Behandlungsfläche 24 absorbiert wird, und im Zuge der Behandlung aufgrund des Verdunstens des dort befindlichen Wassers oder der dort befindlichen Flüssigkeit die Absorption geringer wird, nimmt das Ausgangssignal des Sensors zu.

Aus der Zunahme des Ausgangssignals kann auf das Fortschreiten der Behandlung geschlossen werden. Beispielsweise kann eine Zunahme um 3% bedeuten, dass 1 Millimeter Eindringtiefe nunmehr frei von vitalen Bakterien ist, und entsprechend eine Zunahme entsprechend dem Lambert-Beer-Gesetz $I(x)=I_0{*}e^{-k{*}x}$ , dass die Eindringtiefe nun entsprechend erhöht ist.

Gemäß Fig. 2 ist der Sensor 30 außerhalb der optischen Achse des Lasers 10 angeordnet. Hierdurch wird auch die Streuung der Laserstrahlung im Zahn 25 berücksichtigt.

Gemäß Fig. 3 ist der Laser in der optischen Achse angeordnet, so dass Laserstrahlung nach entsprechender Absorption unmittelbar auf den Sensor 30 trifft.

Die Sensoren 30 gemäß den Figuren 2 und 3 sind als Transmissionssensoren ausgebildet. Alternativ ist es auch möglich, einen Reflexionssensor zu realisieren.

Ein derartiger Reflexionssensor ist unmittelbar auf die Behandlungsfläche 24 gerichtet. Der Reflexionssensor erfasst die Trocknung der Behandlungsfläche durch die Laserstrahlung, da eine feuchte Behandlungsfläche 24 stärker reflektiert als eine getrocknete.

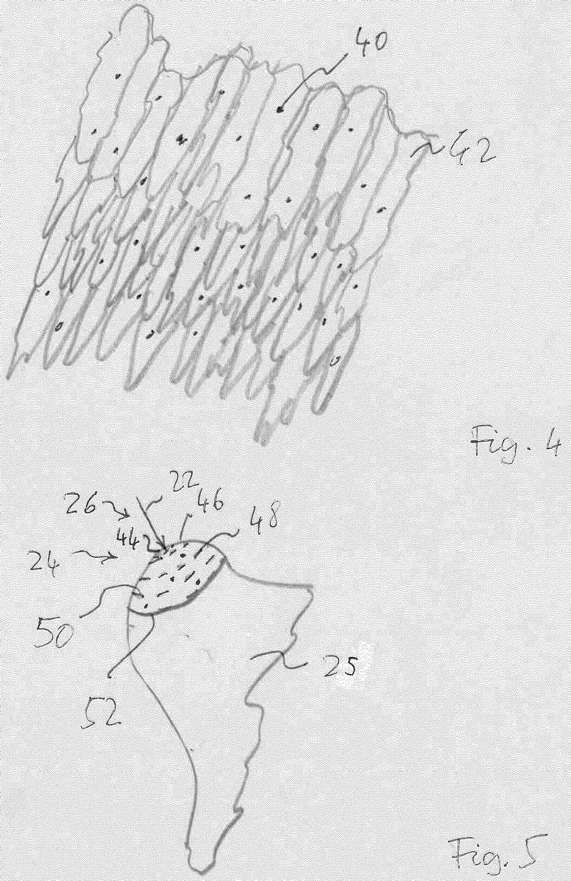

Aus Fig. 4 ist beispielhaft die Einlagerung von Kariesbakterien 40 in ein Hartgewebe 42 ersichtlich. Im Beispielsfall ist das Hartgewebe Zahnschmelz, in den sowohl Flüssigkeit als auch Bakterien eindringen können. Die Kristallstruktur des Zahnschmelzes erlaubt es den Kariesbakterien, sich in drei Dimensionen zu verteilen, wobei in aller Regel das Eindringen an der Zahnoberfläche beginnt.

Typischerweise ist die Bakteriendichte dementsprechend an der Zahnoberfläche höher, aber für eine erfolgreiche Kariesbeseitigung ist es erforderlich, auch die wenigen tiefliegenden Bakterien mindestens so weit zu schädigen, dass sie nicht mehr fortpflanzungsfähig sind. Dies lässt sich mit dem erfindungsgemäßen Bakterienentfernungslaser realisieren.

Die in den Figuren 2 und 3 dargestellten Sensoren 30 können bei der Wellenlänge des Lasers 10 messen. Alternativ und bevorzugt sind sie an einer beliebigen geeigneten Stelle angeordnete Temperatursensoren 30, die in Impulspausen und/oder bei Wellenlängen oberhalb oder unterhalb der Laserwellenlänge messen. Die Temperatursensoren können an einer beliebigen geeigneten Stelle angeordnet sein.

Geeignete preisgünstige Temperatursensoren erfassen Wellenlängen zwischen 1200 und 2500 nm.

Aus Fig. 5 ist ersichtlich, in welcher Weise die Beaufschlagung mit den Bakterien schädigenden Wellenlängen fortschreitet. Zunächst wird eine erste Schicht 44 unmittelbar angrenzend an die Behandlungsfläche 24 von dem Laserstrahl 22 getroffen. Dort bestehende Bakterien werden geschädigt.

Das Schädigen kann so erfolgen, dass die Zellwände zerstört werden, wobei aber auch eine beliebige andere Entfernung möglich ist, beispielsweise auch eine vollständige Entfernung der in der Zelle bestehenden Flüssigkeit durch die Laserstrahlung.

Sobald der Feuchtigkeitsgrad der ersten Schicht 44 deutlich reduziert ist, gelangt ein großer Teil der Laserstrahlung 22 in den Bereich der zweiten Schicht 46. Dies wird auch mit der dritten, vierten und fünften Schicht 48, 50 und 52 so wiederholt, bis die Bakterien im gesamten Bereich der Karies 26 vernichtet oder geschädigt sind.

Die Steuervorrichtung 21 steuert die Strahlungsquelle 12 so, dass zunächst in den oberen Schichten des Zahnes 25 die Kariesbakterien geschädigt werden. Diese erwärmen sich und trocknen, so dass der Temperaturanstieg dort bei gleicher Laserleistung größer wird.

Bevorzugt wird die Strahlungsquelle 12 gepulst betrieben. Zudem wird sie periodisch ein- und ausgeschaltet. z.B. alle 3 Sekunden.

Nach einer gewissen Zeit sind sämtliche Kariesbakterien in der Schicht 44 vernichtet. Ab diesem Zeitpunkt werden schwerpunktmäßig die der zweiten Schicht 46 entfernt, usw.

Die Temperatur des Zahnes 25, insbesondere Behandlungsfläche 24, wird vom Sensor 30 permanent gemessen, oder aber von einem weiteren Sensor.

Wenn der Temperaturanstieg einen Schwellenwert übersteigt, bedeutet dies, dass die Kariesbakterien dort entfernt sind. Dies lässt sich aus dem Temperaturgradienten entnehmen, der von der Steuervorrichtung 21 überwacht wird.

Nachfolgend sind beispielhafte Betriebsdaten eines erfindungsgemäßen Bakterienentfernungslasers 10 aufgestellt:

| Laser Er:YAG, Die Wellenlänge des Laserlichtes beträgt 2940 nm | |
|---|---|
| 0,26 mJ/mm$^2$ | - Energiedichte gemittelt |
| 50 - 500 Hz | - Frequenz Laserpulse |
| 3 - 5 mm | - Behandlungsfläche |
| 1 - 20 min | - Bestrahlungsdauer |
| 21,7 W | - Impulsmaximalleistung |
| 30 - 300 μs | - Impulsbreite |
| 0,5 - 2 W | - mittlere Laserleistung |
| Keine Vorbehandlung, kein Bohren erforderlich | |

In einer weiteren erfindungsgemäßen Ausführungsform wurden die folgenden Betriebsdaten verwendet:

**Laser Parameter**

| | | |
|---|---|---|
| Wellenlänge | 2,94 | μm |
| I | 300 | A |
| Frequenz | 150 | Hz |
| tp | 150 | μs |
| Durchmesser Fokus | 4 | mm |
| Fläche | 12,6 | mm$^2$ |
| Laser Energie | 0,72 | W |

### Energiedichte

| | | |
|---|---|---|
| Periode | 6,67 | ms |
| Duty Cycle | 2,25 | % |
| Peak Power | 0,0048 | W*s |
| Peak Power pro Fläche | 0,000382 | J / mm$^2$ |
| | 0,3820 | mJ / mm$^2$ |

### Energiedichte

| | | |
|---|---|---|
| Energiedichte | 0,00038197 | J / mm$^2$ |
| | 0,382 | mJ / mm$^2$ |

### Peak Power

| | | |
|---|---|---|
| Peak Power | 32 | W*s |

### Energie per Peak

| | | |
|---|---|---|
| Energie per Peak | 0,0048 | J |

### Leistungsdichte

| | | |
|---|---|---|
| Leistungsdichte | 0,0573 | W/mm$^2$ |

### Durchschnittsleistung

| | | |
|---|---|---|
| Paverag (on full Period) | 0,72 | W |

Ferner wurde die theoretische Entfernungs- oder Tötungsrate der Kariesbakterien berechnet: Ihr Durchmesser liegt zwischen etwa 0,1 und 700 $\mu$m, bei den meisten bekannten Arten beträgt er etwa 0,6 bis 1,0 $\mu$m. Ihre Länge liegt in einem größeren Bereich: bei Einzelzellen zwischen etwa 0,6 $\mu$m (bei Kokken) und 700 $\mu$m, Hyphen können noch länger sein, die meisten Bakterien sind 1 bis 5 $\mu$m lang. Das Volumen der meisten Bakterien liegt in der Größenordnung von 1 $\mu$m$^3$.

### Annahme Bakterium

| | | |
|---|---|---|
| Volumen | 1 | $\mu$m$^3$ |
| Radius | 0.620 | $\mu$m |
| Durchmesser | 1,24 | $\mu$m |
| Dichte Wasser | 1 | g/cm$^3$ |
| Masse | 1E-12 | g |
| Masse Bakterium | 1.0E-06 | $\mu$g |
| Temperatur | 298 | K |
| Hv | 44.0 | kJ/mol |
| 100 % Wasser 10$^{-6}$ $\mu$g | 2.45E-09 | J |
| Mit 1 W | **408'869'870** | **Bakterien / Sekunde** |

Das heißt, dass rund 2,5E-09 W*s = 2,5E-09 Joule erforderlich sind, um ein Bakterium zu verdampfen. Annahme: 100 % Wasser und 100 % der Energie geht in die Verdampfungsenthalpie. Somit könnte man mit 1 Watt rund 400 Millionen Bakterien pro Sekunde verdampfen.

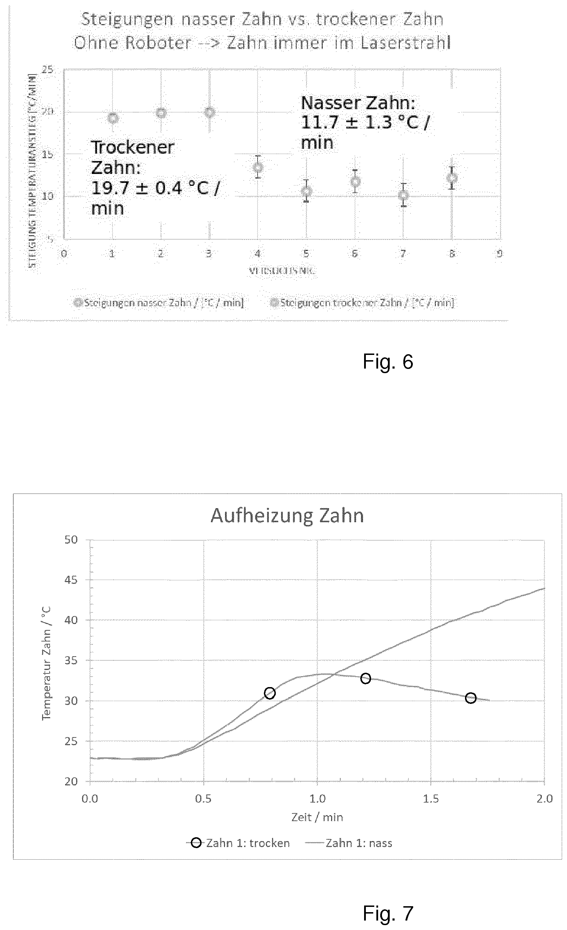

In Fig. 6 ist der gemessene Temperaturanstieg beim Betrieb des Bakterienentfernungslasers 10 im Vergleich zwischen "nass" und "trocken" dargestellt.

Ein trockener Zahn wurde genommen und mit dem typischen Laserparameter (siehe oben) beaufschlagt. Gleichzeitig wurde die Temperatur gemessen. Die Aufheizrate (Steigung) in Grad pro Minute in der ersten Minute nach dem Einschalten wurde anhand eines linearen Fits bestimmt und in Fig. 6 dargestellt. Der trockene Zahn zeigt eine schnellere Erhitzung, weil keine Energie für die Verdampfung des Wassers benötigt wird. Dieser Effekt kann für eine intelligente, individuelle Bestimmung für das Ende der Behandlung benutzt werden, wie sie in Anspruch 8 beansprucht ist.

Fig. 7 zeigt die Aufheizkurve eines Zahnes. Hier wird der Temperaturanstieg eines nassen Zahnes sowie eines trockenen dargestellt.

Es ist ersichtlich, dass der zeitliche Temperaturgradient des trockenen Zahnes deutlich höher ist, wobei die Energiebeaufschlagung nach ca. einer Minute abgebrochen wurde, nachdem die Auswertung ergeben hat, dass dort sämtliche Nässe - also auch Bakterien - verschwunden sind.

In einer weiteren bevorzugten Ausgestaltung ist parallel zu dem Laserstrahl ein Zielstrahl mit sichtbarem Licht vorgesehen. Durch diesen ist für den Zahnarzt sichtbar, wo er behandelt.

**Patentansprüche**

1. Bakterienentfernungslaser, insbesondere zur Entfernung von Kariesbakterien an natürlichen oder prothetischen Zähnen, letzteres auch extraoral; mit einer Greifhandhabe, mit einer Laser-Strahlungsquelle, mit einer Beaufschlagungsoptik mit einer vorgegebenen Strahlungsaustrittsfläche, insbesondere einem Strahlungsleitstab, die von Laserstrahlung durchtreten wird, und mit einer Energiequelle wie einem Netzanschluss oder einem Akkumulator, **dadurch gekennzeichnet, dass** der Laser im Wellenlängenbereich zwischen 2200 nm und 4000 nm emittiert und insbesondere als Er:YAG-Laser ausgebildet ist, mit einer über die Zeit gemittelten Energieabgabe von weniger als 1 mJ/mm$^2$ an der Strahlungsaustrittsfläche.

2. Bakterienentfernungslaser nach Anspruch 1, **dadurch gekennzeichnet, dass** er einen Sensor aufweist, der auf Strahlung im Wellenlängenbereich zwischen 1000 nm und 15000 nm anspricht und insbesondere sein Empfindlichkeitsmaximum oberhalb oder unterhalb des Emissionsmaximums des Lasers hat.

3. Bakterienentfernungslaser nach Anspruch 2, **dadurch gekennzeichnet, dass** der Sensor als Reflexionserfassungssensor ausgebildet ist und, und von einer Behandlungsfläche reflektierte Strahlung mit dem Sensor erfassbar ist und dass die reflektierte Strahlung insbesondere die Beaufschlagungsoptik durchtritt.

4. Bakterienentfernungslaser nach Anspruch 2, **dadurch gekennzeichnet, dass** der Sensor als Transmissionserfassungssensor ausgebildet ist und Strahlung - ggf. nach Umlenkung - erfasst, die eine Behandlungsfläche durchtritt.

5. Bakterienentfernungslaser nach einem der vorhergehenden Ansprüche, **dadurch gekennzeichnet, dass** ein Sensor des Lasers die Temperatur einer Behandlungsfläche in situ erfasst und insbesondere, dass bei einer Temperatur von mehr als 55 Grad C, insbesondere von mehr als 45 Grad C, mit einer Steuervorrichtung für den Laser, dessen Energieabgabe verminderbar oder auf null reduzierbar ist.

6. Bakterienentfernungslaser nach einem der vorhergehenden Ansprüche, **dadurch gekennzeichnet, dass** mit der Laserstrahlungsquelle gepulste Laserstrahlung mit einem Impüls/Pausen-Verhältnis zwischen 1:1 und 1:1000, insbesondere zwischen 1:5 und 1:200 und bevorzugt etwa 1:25 abgebbar ist, mit einer über die Zeit gemittelten Leistung von weniger als 2 W, besonders bevorzugt von etwa 0,8 Watt.

7. Bakterienentfernungslaser nach einem der vorhergehenden Ansprüche, **dadurch gekennzeichnet, dass** mit der Laserstrahlungsquelle gepulste Laserstrahlung mit einer Periodendauer zwischen 0,02 s und 0,002 s abgebbar ist, mit einer gemittelten Leistung von weniger als 2 W, und einer Schaltvorrichtung für das Ein- und Ausschalten der Laserstrahlungsquelle mit einer Frequenz von weniger als 1 Hz, bevorzugt etwa 0,3 Hz, und/oder einer Behandlungsflächen-Schutzvorrichtung, z.B. einer Irisblende oder einem Spiegel, der vor die Strahlungsaustrittsfläche bewegbar ist und die Behandlungsfläche vor der Strahlung schützt, mit welcher die Behandlungsfläche für eine vorgegebene oder einstellbare oder regelbare Zeit vor der Strahlung schützbar ist.

8. Bakterienentfernungslaser nach einem der vorhergehenden Ansprüche, **dadurch gekennzeichnet, dass** eine Steuervorrichtung des Bakterienentfernungslasers an einen Temperatursensor angeschlossen ist, mit welchem die Temperatur der Behandlungsfläche messbar ist, und der Bakterienentfernungslaser abschaltbar ist, wenn der gemessene

Temperaturanstieg $\left( \dfrac{\Delta T}{t} \right)$ größer als ein vorgegebener Schwellenwert ist, der insbesondere größer als der Temperaturanstieg bei einem nassen Behandlungsvolumen und höchstens so groß wie der Temperaturanstieg bei einem trockenen Behandlungsvolumen ist.

9.   Bakterienentfernungslaser nach einem der vorhergehenden Ansprüche, **dadurch gekennzeichnet, dass** mit einem Sensor reflektierte, transmittierte oder emittierte Strahlung der Behandlungsfläche in Impulspausen erfassbar ist.

10.  Bakterienentfernungslaser nach einem der vorhergehenden Ansprüche, **dadurch gekennzeichnet, dass** die Strahlungsaustrittsfläche und/oder die Behandlungsfläche einen Durchmesser zwischen 3 und 5 mm aufweist und/oder dass der Bakterienentfernungslaser eine Lichtquelle mit sichtbarem Licht aufweist, die zusätzlich zur Laser-Strahlungsquelle emittiert und mit wieder sichtbares Licht durch die Strahlungsaustrittsfläche leitbar ist und insbesondere auf die Behandlungsfläche werfbar ist und diese erleuchtbar ist.

11.  Bakterienentfernungslaser nach einem der vorhergehenden Ansprüche, **dadurch gekennzeichnet, dass** dem Laser eine Sterilwasserquelle zugeordnet ist, mit welcher nach dem Ausschalten des Lasers - aber nicht während des Einschaltzustands des Lasers - die Behandlungsfläche mit sterilem Wasser benetzbar ist.

## Claims

1.   A bacteria removal laser, in particular for the removal of caries bacteria on natural or prosthetic teeth, the latter also being extra-orally, with a gripping handle, comprising a laser radiation source, with an optical system with a predetermined beam exit surface, in particular a radiation guide rod, through which laser radiation passes, and having an energy source such as a mains connection or an accumulator, **characterized in that** the laser emits in the wavelength range between 2200 nm and 4000 nm, and in particular is formed as a Er:YAG laser, having an energy output, averaged over time, of less than 1 mJ/mm$^2$ at the radiation exit surface.

2.   The bacteria removal laser according to claim 1, **characterized in that** it comprises a sensor which responds to radiation in the wavelength range between 1000 nm and 15000 nm and in particular has its sensitivity maximum above or below the emission maximum of the laser.

3.   The bacterial removal laser according to claim 2, **characterized in that** the sensor is designed as a reflection detection sensor, and radiation reflected from a treatment surface can be detected by the sensor and **in that** the reflected radiation in particular passes through the impingement optics.

4.   The bacterial removal laser according to claims 2, **characterized in that** the sensor is designed as a transmission detection sensor and, and radiation - optionally after deflection - which passes through a treatment surface is detectable by the sensor.

5.   The bacteria removal laser according to one of the preceding claims, **characterized in that** a sensor of the laser detects the temperature of a treatment surface in situ and, in particular, **in that** at a temperature of more than 55 degrees C, in particular of more than 45 degrees C, a control device for the laser is provided, the energy output of which is reducible to or is reduced to zero.

6.   The bacterial removal laser according to one of the preceding claims, **characterized in that** laser radiation pulsed with the laser radiation source can be emitted with a pulse/pause ratio between 1:1 and 1:1000, in particular between 1:5 and 1:200 and preferably about 1:25, with a power averaged over time of less than 2 W, particularly preferably of about 0.8 Watt.

7.   The bacteria removal laser according to one of the preceding claims, **characterized in that** laser radiation pulsed with the laser radiation source is emittable with a period duration between 0.02 s and 0.002 s, comprising an averaged power of less than 2 W, and a switching device for switching the laser radiation source on and off with a frequency of less than 1 Hz, preferably about 0.3 Hz, and/or a treatment surface protection device, e.g. an iris diaphragm or a mirror which is movable in front of the radiation exit surface, protecting the treatment surface from the radiation, with which the treatment surface is protectable from the radiation for a predetermined or adjustable or controllable time.

8. The bacteria removal laser according to one of the preceding claims, **characterized in that** a control device of the bacteria removal laser is connected to a temperature sensor with which the temperature of the treatment surface can be measured, and the bacteria removal laser can be switched off if the measured temperature rise $\left(\frac{\Delta T}{t}\right)$ in particular is greater than the temperature rise in a wet treatment volume and at most as great as the temperature rise in a dry treatment volume.

9. The bacteria removal laser according to one of the preceding claims, **characterized in that** radiation of the treatment surface which is reflected, transmitted or emitted is detectable by a sensor during pulse pauses.

10. The bacteria removal laser according to one of the preceding claims, **characterized in that** the radiation exit surface and/or the treatment surface has a diameter of between 3 and 5 mm and/or that the bacteria removal laser has a light source with visible light, which emits in addition to the laser radiation source and is guidable across the radiation exit surface and in particular is projectable onto the treatment surface and the latter being illuminatable.

11. The bacteria removal laser according to one of the preceding claims, **characterized in that** the laser is associated with a sterile water source with which the treatment surface is wettable with sterile water after the laser is switched off - but not during the switched-on state of the laser.

## Revendications

1. Laser d'élimination de bactéries, en particulier pour l'élimination de bactéries de caries sur des dents naturelles ou prothétiques, également extra-orales pour ces dernières, avec une poignée de préhension, avec une source de rayonnement laser, avec un système d'application optique avec une surface de sortie de rayonnement prédéterminée, en particulier une barre de guidage de rayonnement, qui est traversée par le rayonnement laser, et avec une source d'énergie telle qu'un raccordement au réseau ou un accumulateur, **caractérisé en ce que** le laser émet dans la plage de longueurs d'onde comprise entre 2200 nm et 4000 nm et en particulier est configuré comme laser Er : YAG, avec un rendement énergétique moyen dans le temps, inférieur à 1 mJ/mm$^2$ à la surface de sortie du rayonnement.

2. Laser d'élimination de bactéries selon la revendication 1, **caractérisé en ce qu'**il comporte un capteur qui réagit à un rayonnement dans la plage de longueurs d'onde comprise entre 1000 nm et 15000 nm et qui présente en particulier son maximum de sensibilité au-dessus ou en dessous du maximum d'émission du laser.

3. Laser d'élimination de bactéries selon la revendication 2, **caractérisé en ce que** le capteur est conçu comme un capteur de détection de réflexion et le rayonnement réfléchi par une surface de traitement peut être détecté par le capteur et **en ce que** le rayonnement réfléchi passe en particulier à travers le système d'application optique.

4. Laser d'élimination de bactéries selon la revendication 2, **caractérisé en ce que** le capteur est conçu comme un capteur de détection de transmission et détecte le rayonnement - éventuellement après déviation - qui passe à travers une surface de traitement.

5. Laser d'élimination de bactéries selon l'une des revendications précédentes, **caractérisé en ce qu'**un capteur du laser détecte in situ la température d'une surface de traitement et en particulier celle à une température supérieure à 55 degrés C, en particulier supérieure à 45 degrés C, avec un dispositif de commande pour le laser, dont la puissance de sortie d'énergie peut être réduite ou ramenée à zéro.

6. Laser d'élimination de bactéries selon l'une des revendications précédentes, **caractérisé en ce que** le rayonnement laser pulsé avec la source de rayonnement laser peut être émis avec un rapport impulsion/pause compris entre 1:1 et 1:1000, en particulier entre 1:5 et 1:200 et de préférence environ 1:25, avec une puissance moyenne dans le temps inférieure à 2 W, en particulier de préférence d'environ 0,8 W.

7. Laser pour l'élimination de bactéries selon l'une des revendications précédentes, **caractérisé en ce qu'**un rayonnement laser pulsé d'une durée de période comprise entre 0,02 s et 0,002 s peut être émis avec la source de rayonnement laser, avec une puissance moyenne inférieure à 2 W, et un dispositif de commutation pour la mise en marche et l'arrêt de la source de rayonnement laser avec une fréquence inférieure à 1 Hz, de préférence environ 0,3 Hz, et/ou un dispositif de protection de la surface de traitement, par exemple, un diaphragme d'iris ou un miroir qui est mobile devant la surface de sortie du rayonnement et qui protège la surface de traitement contre le rayonnement, avec lequel la surface de traitement peut être protégée du rayonnement pendant une durée prédéterminée ou réglable ou contrôlable.

8. Laser d'élimination des bactéries selon l'une des revendications précédentes, **caractérisé en ce qu'**un dispositif de commande du laser d'élimination des bactéries est relié à un capteur de température avec lequel la température de la surface de traitement peut être mesurée, et le laser d'élimination des bactéries peut être $\left(\frac{\Delta T}{t}\right)$ éteint si l'augmentation de température mesurée est supérieure à une valeur seuil prédéterminée qui est en particulier supérieure à l'augmentation de température d'un volume de traitement humide et au maximum équivalent à l'augmentation de température d'un volume de traitement sec.

9. Laser d'élimination des bactéries selon l'une des revendications précédentes, **caractérisé en ce que** le rayonnement de la surface de traitement qui est réfléchi, transmis ou émis peut être détecté par un capteur dans les pauses d'impulsion.

10. laser d'élimination de bactéries selon l'une des revendications précédentes, **caractérisé en ce que** la surface de sortie du rayonnement et/ou la surface de traitement a un diamètre compris entre 3 et 5 mm et/ou **en ce que** le laser d'élimination de bactéries présente une source lumineuse avec de la lumière visible, qui émet en plus de la source de rayonnement laser et peut être guidée à travers la surface de sortie du rayonnement avec de la lumière qui est à nouveau visible et peut être projetée en particulier sur la surface de traitement et peut l'éclairer.

11. Laser d'élimination de bactéries selon l'une des revendications précédentes, **caractérisé en ce qu'**une source d'eau stérile est associée au laser, avec laquelle la surface de traitement peut être mouillée avec de l'eau stérile après l'arrêt du laser - mais pas pendant que le laser est en état de marche du laser.

Fig. 1

Fig. 3

Fig. 2

Fig. 4

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1052947 A1
- US 20120015318 A1